# EUROPÄISCHE PATENTSCHRIFT

(11) **EP 3 852 564 B1**
(45) Veröffentlichungstag und Bekanntmachung des Hinweises auf die Patenterteilung: **31.08.2022**
(21) Anmeldenummer: 18808211.9
(22) Anmeldetag: 29.10.2018
(51) Int. Cl.: A41D 19/04, A41D 19/00, A41D 19/015, A61B 42/50

(54) **FORMKÖRPER ZUR HERSTELLUNG EINES HANDSCHUHS**
FORMER BODY FOR PRODUCING A GLOVE
CORPS MOULÉ POUR LA FABRICATION D'UN GANT

(30) Priorität: 18.09.2018 DE 102018122863
(43) Veröffentlichungstag der Anmeldung: 28.07.2021
(73) Patentinhaber: YTY Industry Sdn. Bhd., 50400 Kuala Lumpur (MY)
(72) Erfinder: DIERS, Paul, 52062 Aachen (DE); GLESER, Maxim, 30880 Laatzen (DE)
(74) Vertreter: Sandvoß, Stefanie
(86) Internationale Anmeldenummer: PCT/DE2018/100883
(87) Internationale Veröffentlichungsnummer: WO 2020/057677

(56) Entgegenhaltungen:
- WO-A1-2016/070868
- US-A- 5 365 608

## Beschreibung

Die Erfindung betrifft einen Formkörper zur Herstellung eines Handschuhs, insbesondere eines Untersuchungs- oder Operationshandschuhs oder eines Arbeitshandschuhs, der eine Greifhilfe aufweist. Weiterhin betrifft die Erfindung auch ein Verfahren zur Herstellung des Formkörpers sowie die Verwendung des Formkörpers zum Herstellen eines Handschuhs.

### Stand der Technik

Es ist bekannt, Handschuhe, z.B. aus Latex oder Nitril, herzustellen, indem ein Formkörper mit der Geometrie einer Hand und einem an die Hand ansetzenden zylindrischen Abschnitt in ein Tauchbad getaucht, anschließend wieder aus dem Tauchbad entfernt und trocknen gelassen wird. Ist das durch das Eintauchen in das Tauchbad aufgenommene und an dem Formkörper anhaftende Material getrocknet, so kann dieses in Form des Handschuhs von dem Formkörper abgestreift werden, beispielsweise manuell oder maschinell. Der Formkörper kann schließlich erneut zur weiteren Herstellung von Handschuhen verwendet werden.

Ein solches gattungsgemäßes Verfahren wird beispielsweise durch die MY 165912 A beschrieben.

In der DE 199 52 480 A1 wird ein ähnliches Verfahren beschrieben, das jedoch einen zusätzlichen Schritt aufweist. Hier wird nach dem Eintauchen der Formkörper in ein Tauchbad zum Erzeugen einer Materialschicht eine zweite Schicht auf die Materialschicht aufgebracht, z.B. ebenfalls mittels Eintauchens in ein Tauchbad.

Die WO 2016/058029 A1 beschreibt ebenfalls ein gattungsgemäßes Tauchverfahren zur Herstellung von Handschuhen mittels Formkörpern, wobei die Formkörper jeweils im Bereich der Fingerspitzen Strukturierungen aufweisen und die entsprechenden mittels der Formkörper hergestellten Handschuhe ebenfalls an den Fingerspitzen Strukturierungen aufweisen.

Ein Verfahren zur Herstellung von Handschuhen, die eine Greifhilfe mit Strukturierungen sowie Fingerspitzen mit Strukturierungen aufweisen, wird durch die WO 2016/070868 A1 beschrieben. Auch diese Handschuhe werden durch Eintauchen von Formkörpern in ein Tauchbad hergestellt, weshalb die verwendeten Formkörper eine Greifhilfe mit Strukturierungen sowie auch Strukturierungen im Bereich der Fingerspitzen aufweisen.

Ein Problem bei der Herstellung von Handschuhen mit Greifhilfe, das sich gezeigt hat, liegt darin, dass beim Eintauchen der ebenfalls eine Greifhilfe aufweisenden Formkörper in das Tauchbad die Materialanhaftung am Formkörper durch die Greifhilfe nicht an allen Stellen ausreichend ist bzw. die Greifhilfe nach dem Entfernen der Formkörper aus dem Tauchbad bewirkt, dass das Material nicht wie bei Handschuhen ohne Greifhilfe in Richtung der Fingerspitzen am Formkörper hinunterfließen und abtropfen kann, sondern durch die Greifhilfe zum Teil umgeleitet wird, was zu einer inhomogenen Materialverteilung führen kann. An Stellen mit geringer Materialanhaftung können daher beim bzw. nach dem Trocknen kleine Löcher auftreten, die natürlich dazu führen, dass der Handschuh nicht mehr zu verwenden ist.

### Aufgabe der Erfindung

Gegenüber dem bekannten Stand der Technik ist es die Aufgabe der vorliegenden Erfindung, einen alternativen Formkörper zur Herstellung eines Handschuhs mit Greifhilfe bereitzustellen, der bevorzugt die aus dem Stand der Technik bekannten Nachteile in der Herstellung der Handschuhe vermeidet und sich insbesondere dadurch auszeichnet, dass beim Eintauchen des Formkörpers in ein Tauchbad und beim anschließenden Trocknen eine an allen Stellen ausreichende Materialanlagerung erfolgt. Weiterhin soll ein alternatives Verfahren zur Herstellung eines entsprechenden Formkörpers bereitgestellt werden.

### Allgemeine Beschreibung der Erfindung

Die Erfindung löst die Aufgabe mit den Merkmalen der Ansprüche und insbesondere durch einen Formkörper zur Herstellung eines Handschuhs, wobei der Formkörper die Geometrie einer Hand mit einem daran ansetzenden im Wesentlichen zylindrischen Abschnitt und mit einer Greifhilfe aufweist, die unterhalb des kleinen Fingers oder unterhalb des Daumens an einer der beiden die Handfläche und den Handrücken verbindenden Schmalseiten der Hand oder alternativ auf der Verlängerung einer dieser Schmalseiten der Hand in Längsrichtung des mit der Hand verbundenen zylinderförmigen Abschnitts angeordnet ist. Erfindungsgemäß weist der Formkörper dabei einen Bereich mit strukturierter Oberfläche am Ansatz der Greifhilfe auf, der sich vorzugsweise in Richtung der Finger der Hand erstreckt und stärker strukturiert ist als andere Bereiche des Formkörpers, wobei der Formkörper vorzugsweise auch auf der Greifhilfe sowie an zumindest zwei Fingerspitzen der Hand jeweils zumindest einen Bereich mit strukturierter Oberfläche aufweist, und wobei auch diese Bereiche mit strukturierter Oberfläche auf der Greifhilfe sowie an zumindest zwei Fingerspitzen der Hand vorzugsweise stärker strukturiert sind als andere Bereiche des Formkörpers.

Mit "Ansatz" der Greifhilfe ist das der Spitze der Greifhilfe gegenüberliegende Ende der Greifhilfe gemeint, das unmittelbar in den zylinderförmigen Abschnitt übergeht, der zur Formgebung der Stulpe von mit dem Formkörper hergestellten Handschuhen vorgesehen ist. Der Ansatz der Greifhilfe liegt dabei auf einer gedachten Linie, die beim Blick von vorne auf den mit nach oben weisenden Fingerspitzen angeordneten Formkörper eine Seite des zylinderförmigen Abschnitts begrenzt und parallel ist zu der um 180 ° beabstandeten Seite, die gegenüberliegend den zylinderförmigen Abschnitt begrenzt.

Erfindungsgemäß muss nicht der gesamte Ansatz der Greifhilfe strukturiert sein, sondern es reicht, wenn sich ein strukturierter Bereich am Ansatz befindet, während der restliche Teil des Ansatzes unstrukturiert oder weniger stark strukturiert ist.

Der Bereich am Ansatz der Greifhilfe erfindungsgemäßer Formkörper muss nicht notwendigerweise der Bereich sein, der am stärksten strukturiert ist. Beispielsweise können die vorzugsweise vorhandenen strukturierten Bereiche an den Fingerspitzen und/oder der vorzugsweise vorhandene strukturierte Bereich auf der Greifhilfe stärker strukturiert sein als der Bereich am Ansatz der Greifhilfe. Die Formulierung, dass der Bereich am Ansatz der Greifhilfe stärker strukturiert ist als andere Bereiche des Formkörpers bedeutet daher nicht, dass dieser Bereich stärker strukturiert ist als alle anderen Bereiche des Formkörpers, sondern nur, dass der Formkörper Bereiche aufweist, die weniger stark oder gar nicht strukturiert sind. Insbesondere grenzt ein Bereich mit weniger starken oder gar keinen Strukturierungen auf der der Greifhilfe gegenüberliegenden Seite des strukturierten Bereiches am Ansatz der Greifhilfe an diesen an, wobei dieser Bereich mit weniger starken oder gar keinen Strukturierungen insbesondere auf einer gedachten Linie angeordnet ist, die parallel zu dem den Fingerspitzen gegenüberliegenden Ende des Formkörpers verläuft.

Bei aus dem Stand der Technik bekannten Handschuhen mit seitlich des Handgelenks angeordneter Greifhilfe zeigte sich oftmals das Problem, dass in dem als "crotch area" bezeichneten Bereich zwischen der Greifhilfe und dem Fingerraum, der an einen Daumen angepasst ist, kleine Löcher auftraten, deren Entstehung darauf zurückgeführt wurde, dass die Materialanhaftung in der "crotch area" beim Eintauchen der Formkörper in ein Tauchbad und beim anschließenden Trocknen nicht ausreichend ist, da Material aus dem Tauchbad von der Greifhilfe abgefangen wurde. Dies ist hier problematischer als beispielsweise im Bereich der Finger bzw. der Ansätze der Finger, da die Materialdicke der Handschuhe in Richtung der Fingerspitzen zunimmt.

Zur Lösung des Problems wurden verschiedene Ansätze verfolgt, die jedoch nicht vollständig zufriedenstellende Lösungen brachten. Beispielsweise wurden auf den Formern Flüssigkeitsreservoire vorgesehen, in denen sich beim Eintauchen der Formkörper Flüssigkeit aus dem Tauchbad ansammelte, die nach dem Herausnehmen der Formkörper aus dem Tauchbad durch gezielte Dreh-/ und/oder Kippbewegungen der Formkörper in die crotch area fließen sollte.

Weiterhin wurden auf der Greifhilfe selbst Bereiche mit strukturierter Oberfläche angeordnet, wobei durch die gezielte Anordnung von Noppen oder anderen strukturierten Oberflächen auf der Greifhilfe erreicht werden sollte, dass das an den Formkörpern anhaftende Material aus dem Tauchbad während des Abtropf- bzw. Trocknungsschritts gezielt umverteilt wird. Auch dies führte jedoch nicht dazu, dass der Ausschuss an Handschuhen mit seitlich des Handgelenks angeordneter Greifhilfe aufgrund von kleinen Löchern in der crotch area erheblich zurückging.

Denn es wurde gefunden, dass das eigentliche Problem bei der Herstellung von Handschuhen mit seitlich des Handgelenks angeordneter Greifhilfe weniger in der geringen Materialstärke im Bereich der crotch area liegt, sondern daran, dass die in der Herstellung verwendeten Formkörper durch die Greifhilfe schlechter zu reinigen sind als Formkörper ohne Greifhilfe. Mit der Zeit haften daher kleinere Partikel leicht in der crotch area am Formkörper an, wodurch die Oberfläche in diesem Bereich rauer wird und noch schwerer zu reinigen ist. Diese Anhaftungen begünstigt jedoch die Bildung kleiner Löcher, sog. "pin holes", in der crotch area.

Für die Zwecke der Erfindung wird unter der Bezeichnung "crotch area" insbesondere ein Bereich am Ansatz der Greifhilfe verstanden, der sich in Richtung der Fingerspitzen des Formkörpers erstreckt, z.B. um 0,5-3 cm.

Erst mittels der erfindungsgemäßen Formkörper, die einen Bereich mit strukturierter Oberfläche am Ansatz der Greifhilfe aufweisen, ist die Herstellung von Handschuhen mit seitlich des Handgelenks angeordneter Greifhilfe ohne signifikanten Ausschuss möglich. Denn durch den Bereich mit strukturierter Oberfläche am Ansatz der Greifhilfe des Formkörpers wird die Oberfläche in diesem Bereich "versiegelt", die Rauheit steigt und die Anhaftung von Flüssigkeit aus dem Tauchbad an dem Formkörper wird verbessert.

Erfindungsgemäß kann der Formkörper an seiner gesamten Oberfläche oder nur stellenweise Strukturierungen aufweisen. In Ausführungsformen, in denen der Formkörper nur stellenweise Strukturierungen aufweist, befinden sich Bereiche mit strukturierter Oberfläche vorzugsweise auf der Greifhilfe, an zumindest zwei Fingerspitzen des Formkörpers sowie am Ansatz der Greifhilfe. Der Bereich mit strukturierter Oberfläche am Ansatz der Greifhilfe erfüllt dabei die Funktion, dass die mittels des Formkörpers hergestellten Handschuhe eine ausreichende und gleichmäßige Materialdicke in der crotch area aufweisen. Ohne Strukturierungen in diesem Bereich entstehen beim Eintauchen der Formkörper in ein Tauchbad leicht Luftblasen, die platzen, wodurch es zu den sogenannten "pinholes" oder zu einer ungleichmäßgen Materialdicke in der crotch area der erzeugten Handschuhe kommt, was wiederum dazu führen kann, dass die Handschuhe in diesem Bereich beim Ausziehen leicht reißen.

Die vorzugsweise ebenfalls vorhandenen Bereiche mit strukturierter Oberfläche an den Fingerspitzen sowie an der Greifhilfe führen hingegen dazu, dass die mittels des Formkörpers hergestellten Handschuhe ebenfalls an der Greifhilfe sowie an den Fingerspitzen strukturierte Oberflächen aufweisen. Dies ermöglicht ein sicheres Ausziehen der Handschuhe, da die strukturierten Oberflächen an Greifhilfe und Fingerspitzen beim Entfernen der Handschuhe von der Hand interagieren, sodass eine Kontaminationsgefahr durch ein Abrutschen während des Ausziehvorgangs minimiert wird.

Der optionale Bereich mit strukturierter Oberfläche an der Greifhilfe des Formkörpers kann sich dabei erfindungsgemäß über die gesamte Oberfläche der Greifhilfe erstrecken, oder alternativ auch nur auf die Spitze der Greifhilfe sowie den angrenzenden Bereich erstrecken und eine Fläche von ca. 1 bis 2 cm² aufweisen.

Optional weist der Formkörper abgesehen von den Bereichen mit strukturierter Oberfläche am Ansatz der Greifhilfe, an der Greifhilfe sowie an zumindest zwei Fingerspitzen keine weiteren Bereiche mit strukturierter Oberfläche auf. Alternativ kann der Formkörper jedoch auch beliebig viele weitere Bereiche mit strukturierter Oberfläche aufweisen oder sogar vollständig strukturiert sein.

Der Bereich mit strukturierter Oberfläche am Ansatz der Greifhilfe erstreckt sich vorzugsweise ca. 0,1 bis 3 cm, insbesondere 0,5 bis 3 cm, z.B. 1 cm, 2 cm oder 3 cm, in Richtung der Finger des Formkörpers, insbesondere in Form einer ovalen oder parabelförmigen Fläche, die insbesondere eine im Vergleich zu einem angrenzenden Bereich des Formkörpers mit gleichem Abstand zu dessen den Fingerspitzen gegenüberliegenden Ende erhöhte Rauheit aufweist. Die strukturierte Oberfläche am Ansatz der Greifhilfe weist dabei z.B. Erhebungen und Vertiefungen auf, die insbesondere regelmäßig angeordnet sind, zum Beispiel insbesondere parallel verlaufende Rillen, Lamellen, Riffelungen oder regelmäßig oder unregelmäßig beabstandete Rauten, Karos oder Noppen. Gemäß einer Ausführungsform erstreckt sich der Bereich mit strukturierter Oberfläche am Ansatz der Greifhilfe alternativ oder zusätzlich zu der vorgenannten Erstreckung in Richtung der Finger des Formkörpers auch in Richtung der Spitze der Greifhilfe und umgibt diese beispielsweise in dem der Spitze gegenüberliegenden unteren Teil vollumfänglich oder teilumfänglich.

Optional gehen der Bereich mit strukturierter Oberfläche am Ansatz der Greifhilfe und der Bereich mit strukturierter Oberfläche an der Greifhilfe bzw. an der Spitze der Greifhilfe auch ineinander über bzw. bilden nicht zwei voneinander getrennte Bereiche mit strukturierter Oberfläche, sondern nur einen Bereich mit strukturierter Oberfläche, der sich von der Spitze der Greifhilfe bis zum Ansatz der Greifhilfe erstreckt, und sich optional noch weiter - beispielsweise ca. 0,5 bis 3 cm - in Richtung der Finger des Formkörpers erstreckt. In solchen Ausführungsformen, in denen der Bereich mit strukturierter Oberfläche am Ansatz der Greifhilfe und der Bereich mit strukturierter Oberfläche an der Greifhilfe bzw. An der Spitze der Greifhilfe ineinander übergehen und daher nur einen Bereich mit strukturierter Oberfläche bilden, kann dieser optional verschiedene Arten von Strukturierungen und/oder verschiedene Strukturierungstiefen aufweisen.

Gemäß einer Ausführungsform des erfindungsgemäßen Formkörpers weist der am Ansatz der Greifhilfe angeordnete Bereich mit strukturierter Oberfläche Strukturierungen auf, die sich in ihrer Rauheit von den Strukturierungen auf der Greifhilfe und/oder von den Strukturierungen an den Fingerspitzen unterscheiden. Bevorzugt befinden sich dabei die stärksten Strukturierungen an den Fingerspitzen sowie optional zusätzlich auf der Greifhilfe, während die Strukturierungen in dem Bereich am Ansatz der Greifhilfe weniger stark sind, d.h. eine geringere Rauheit aufweisen als die Strukturierungen an den Fingerspitzen.

Gemäß einer alternativen Ausführungsform weisen die Strukturierungen an den Fingerspitzen eine größere Rauheit auf als die Strukturierungen auf der Greifhilfe, wobei die Strukturierungen auf der Greifhilfe eine größere Rauheit aufweisen als die Strukturierungen am Ansatz der Greifhilfe.

Es ist bevorzugt, dass der überwiegende Teil des Formkörpers keine Strukturierungen aufweist oder nur sehr leichte Strukturierungen aufweist, da auch die mit dem Formkörper hergestellten Handschuhe größtenteils unstrukturiert bzw. nur sehr leicht texturiert sein sollen, um den Träger des Handschuhs möglichst wenig zu beeinträchtigen. Gemäß einer bevorzugten Ausführungsform weist der erfindungsgemäße Formkörper daher nur am Ansatz der Greifhilfe, im Bereich der Spitze der Greifhilfe und an zumindest zwei Fingerspitzen Bereiche mit Strukturierungen auf, die zu einer erhöhten Rauheit in diesen Bereichen führen, während der restliche Formkörper gar keine oder nur eine sehr leichte Texturierung mit einer entsprechend niedrigeren Rauheit der Oberfläche aufweist.

Unter "Rauheit" wird für die Zwecke der Erfindung die Rauheit von Oberflächenbereichen des Formkörpers verstanden, die quantitativ durch den Mittenrauwert Ra angegeben werden kann. Vorzugsweise beträgt bei einem erfindungsgemäßen Formkörper der Mittenrauwert Ra des Bereichs mit strukturierter Oberfläche am Ansatz der Greifhilfe 6.0 bis 9.0 µm, im Bereich mit strukturierter Oberfläche auf der Greifhilfe 8.0 bis 10 µm und in den Bereichen mit strukturierter Oberfläche an den Fingerspitzen der Hand 6.0 bis 9.0 µm. Gemäß einer Ausführungsform weist daher der Bereich mit strukturierter Oberfläche auf der Greifhilfe einen höheren Mittenrauwert Ra auf als der Bereich mit strukturierter Oberfläche am Ansatz der Greifhilfe und die Bereiche mit strukturierter Oberfläche an den Fingerspitzen der Hand.

Alternativ dazu kann der Mittenrauwert Ra aller Bereiche mit strukturierter Oberfläche an der Greifhilfe, am Ansatz der Greifhilfe und an den Fingerspitzen jedoch auch gleich sein, wobei er insbesondere höher ist als der Mittenrauwert derjenigen Bereichen, die an die Bereiche mit strukturierter Oberfläche angrenzen, und optional auch höher ist als die jeweiligen Mittenrauwerte in allen weiteren Bereichen des Formkörpers.

Gemäß einer Ausführungsform des erfindungsgemäßen Formkörpers weist der Bereich mit strukturierter Oberfläche am Ansatz der Greifhilfe und/oder einer oder mehrere der Bereiche mit strukturierter Oberfläche auf der Greifhilfe oder an den Fingerspitzen der Hand Strukturierungen auf, die in ihrer Form, Tiefe und/oder Verteilung variieren. Strukturierungen, die in ihrer Form und/oder Tiefe variieren, können beispielsweise durch unterschiedliche Verfahren auf den Formkörper aufgebracht worden sein. Hier gibt es grundsätzlich zwei verschiedene Möglichkeiten, Bereiche mit strukturierter Oberfläche auf dem Formkörper herzustellen, nämlich zum einen mittels eines Sandstrahls und zum anderen mittels Aufsprühens von Material, aus dem auch der Formkörper besteht. Dies wird an späterer Stelle im Zusammenhang mit dem erfindungsgemäßen Verfahren zur Herstellung des Formkörpers detaillierter beschrieben.

Bei einer Variation der Verteilung der Strukturierungen in den strukturierten Bereichen am Ansatz der Greifhilfe, an der Spitze der Greifhilfe und an den Fingerspitzen ist es bevorzugt, dass die Dichte der Strukturierungen in der Mitte der strukturierten Bereiche am höchsten ist und in Richtung der Ränder der strukturierten Bereiche abnimmt. Auf diese Weise weisen die Formkörper keinen "harten" Übergang zwischen strukturierten und unstrukturierten bzw. zwischen stärker und schwächer strukturierten Bereichen auf, sondern weisen entsprechend weiche Übergänge zwischen den strukturierten und den unstrukturierten bzw. den stärker und schwächer strukturierten Bereichen auf. Dies hat beim Eintauchen der Formkörper in ein Tauchbad den Vorteil, dass es zu keinem Materialrückstau an den harten Übergängen von strukturierten zu unstrukturierten Bereichen kommt, der in einer abrupten Änderung der Materialdicke resultiert.

Gemäß einer Ausführungsform weist der Formkörper auch einen oder mehrere Bereiche mit Strukturierungen auf, in denen sich unterschiedliche Arten von Strukturierungen, beispielsweise mit unterschiedlichen Formen, Texturierungen und/oder Rauheiten, überlappen. Insbesondere am Ansatz und/oder an der Spitze der Greifhilfe kann dies vorteilhaft sein, da auch auf diese Weise abrupte Übergänge auf dem Formkörper vermieden werden. Die bei Verwendung des Formkörpers erhältlichen Handschuhe weisen somit eine homogene Materialdicke auf, da sich nach dem Eintauchen der Formkörper während des Abtropfens von Material kein Material an den harten Übergängen zwischen strukturierten und unstrukturierten bzw. zwischen stärker und schwächer strukturierten Bereichen ansammelt. Die sich überlappenden unterschiedlichen Arten von Strukturierungen auf dem Formkörper können durch unterschiedliche Verfahren erzeugt sein, z.B. durch eine Sandstrahlbehandlung und durch nachträgliches Aufbringen von Material und/oder können in Form, Tiefe und/oder Verteilung variieren. Beispielsweise kann der am Ansatz der Greifhilfe befindliche Bereich mit strukturierter Oberfläche zwei, drei oder vier sich überlappende Teilbereiche mit unterschiedlich strukturierter Oberfläche aufweisen.

Optional weist der erfindungsgemäße Formkörper an seiner gesamten Oberfläche oder an einem Teil seiner Oberfläche, der insbesondere die Greifhilfe zumindest teilweise einschließt, eine Grundstruktur, z.B. eine Fischschuppen-Struktur auf. Bevorzugt ist die Grundstruktur dabei integraler Bestandteil des Formkörpers und wurde im formgebenden Prozess der Formkörperherstellung direkt mit erzeugt, d.h. zur Formkörperherstellung wurde eine Masse in eine Negativ-Form gegossen, welche die entsprechende Grundstruktur, z.B. die Fischschuppen-Struktur aufwies. Der nach Erhärten der Masse erhaltene Formkörper weist dementsprechend ebenfalls die Grundstruktur an der gesamten Oberfläche oder alternativ nur an ausgewählten Bereichen der Oberfläche auf. Beispielsweise kann der Formkörper nur an der Handinnenfläche eine Grundstruktur aufweisen, um dort insbesondere bei Handschuhen mit hoher Materialdicke die Griffigkeit zu erhöhen. In Fällen, in denen der erfindungsgemäße Formkörper eine Grundstruktur aufweist, kann diese teilweise von Bereichen mit strukturierter Oberfläche überlagert sein, insbesondere im Bereich am Ansatz der Greifhilfe, auf der Greifhilfe und/oder an den Fingerspitzen.

Weiter optional weist der erfindungsgemäße Formkörper an der Greifhilfe eine transparente oder eine farbige, z.B. blaue, Glasierung auf. Bei der Herstellung der Handschuhe mit dem Formkörper geht jeweils ein Teil der Glasierung auf den Handschuh über, d.h. das Material verdunkelt sich an der Stelle der Glasierung. Der Handschuh erhält dadurch eine visuell auffällige Greifhilfe, die eine Signalwirkung entfalten kann und den Träger daran erinnern kann, den Handschuh zum Entfernen von der Hand nur an der Greifhilfe zu erfassen.

Generell ist es bevorzugt, dass der erfindungsgemäße Formkörper Keramik oder eine Keramikmischung umfasst oder daraus besteht, oder ein oder mehrere Polymere umfasst oder daraus besteht. In Fällen, in denen der Formkörper ein Polymer umfasst, ist dieses optional ausgewählt aus der Gruppe, die Polyamide, Polyarylensulfide und Mischungen dieser umfasst.

Die Erfindung betrifft weiterhin auch ein Verfahren zur Herstellung erfindungsgemäßer Formkörper, die einen Bereich mit strukturierter Oberfläche am Ansatz der Greifhilfe aufweisen. In dem erfindungsgemäßen Verfahren werden auf einem Roh-Formkörper mit der Geometrie einer Hand mit einem daran ansetzenden im Wesentlichen zylindrischen Abschnitt und mit einer Greifhilfe, die unterhalb des kleinen Fingers oder unterhalb des Daumens an einer der beiden die Handfläche und den Handrücken verbindenden Schmalseiten der Hand oder alternativ auf der Verlängerung einer dieser Schmalseiten der Hand in Längsrichtung des mit der Hand verbundenen zylinderförmigen Abschnitts angeordnet ist, der Bereich mit strukturierter Oberfläche am Ansatz der Greifhilfe, sowie optional auch Bereiche mit strukturierter Oberfläche auf der Greifhilfe und an zumindest zwei Fingerspitzen der Hand erzeugt. Der verwendete Roh-Formkörper unterscheidet sich dabei dadurch von dem erfindungsgemäßen Formkörper, dass er keine strukturierten Bereiche, sondern eine glatte Oberfläche oder eine Oberfläche mit zumindest anteiliger Grundstruktur aufweist. In Fällen, in denen der Roh-Formkörper eine Grundstruktur aufweist, wurde diese direkt im formgebenden Prozessschritt der Herstellung, d.h. durch Gießen einer Masse in eine Negativ-Form mit entsprechender Oberflächenstruktur und anschließendes Erhärten der Masse in der Form, erzeugt.

Vorzugsweise werden die vorgenannten Bereiche mit strukturierter Oberfläche am Ansatz der Greifhilfe und/oder der Bereich mit strukturierter Oberfläche im Bereich der Spitze der Greifhilfe und/oder die Bereiche mit strukturierter Oberfläche an den Fingerspitzen des Formkörpers mittels einer Sandstrahlbehandlung des Roh-Formkörpers oder dadurch erzeugt, dass zusätzliches Material auf den Bereich bzw. die Bereiche des Roh-Formkörpers aufgetragen, insbesondere aufgesprüht wird.

Damit nicht die gesamte Oberfläche des Formkörpers die gleiche Strukturierungsart und - rauheit erhält, wird der Roh-Formkörper vorzugsweise vor der Sandstrahlbehandlung bzw. vor dem Aufsprühen von Material in einer Schutzhülle angeordnet, die in den Bereichen, in denen Strukturierungen generiert werden sollen, Ausnehmungen aufweisen, beispielsweise am Ansatz der Greihilfe, im Bereich der Spitze der Greifhilfe und/oder an den Fingerspitzen des Formkörpers. Diese Ausnehmungen, d.h. die nicht von der Schutzhülle umgebenen Flächen des Roh-Formkörpers, werden anschließend mittels eines Sandstrahls behandelt. Dabei kann über die Partikelform und -größe der Sandkörner die Geometrie der erzeugten Strukturierungen beeinflusst werden. Alternativ kann auch Material an den nicht von der Schutzhülle umgebenen Flächen des Formkörpers aufgetragen, insbesondere aufgesprüht werden.

Eine mittels Sandstrahl erzeugte strukturierte Fläche des fertigen Formkörpers erzeugt bei der Verwendung des Formkörpers zur Handschuhherstellung Handschuhe, bei denen die Strukturierungen an der Außenoberfläche des Handschuhs angeordnet sind. Denn durch die Sandkörner werden kleine Mulden in den Formkörper geschlagen, in denen sich beim Eintauchen der Formkörper in ein Tauchbad vermehrt Flüssigkeit ansammelt. Die Formkörper werden anschließend aus dem Tauchbad entfernt und trocknen gelassen, und dann werden die fertigen Handschuhe von den Formkörpern abgezogen, wobei die Handschuhe einmal "auf links" gedreht werden.

Im Gegensatz dazu erzeugt eine mittels Aufbringens von Material auf den Roh-Formkörper strukturierte Fläche bei der Verwendung des fertigen Formkörpers zur Handschuhherstellung Handschuhe, bei denen die Strukturierungen an der Innenoberfläche des Handschuhs angeordnet sind. Denn durch das Aufbringen, z.B. Aufsprühen von Material auf Bereiche des Formkörpers werden Erhebungen auf dessen Oberfläche erzeugt, an denen sich beim Eintauchen der Formkörper in das Tauchbad vermehrt Flüssigkeit ansammelt. Die Formkörper werden anschließend aus dem Tauchbad entfernt und trocknen gelassen, und dann werden die fertigen Handschuhe von den Formkörpern abgezogen, wobei die Handschuhe einmal "auf links" gedreht werden, sodass die Erhebungen nach innen weisen.

Die Bereiche mit strukturierter Oberfläche auf dem erfindungsgemäßen Formkörper können in einem oder optional in mehreren Schritten erzeugt werden. Bei der Erzeugung in einem Schritt wird nur einmal eine Schutzhülle mit Ausnehmungen über dem Roh-Formkörper angeordnet, bevor die Behandlung mittels Sandstrahl erfolgt oder Material aufgetragen wird. Bei einer mehrschrittigen Erzeugung von Bereichen mit strukturierter Oberfläche werden mehrmals Schutzhüllen auf dem Roh-Formkörper angeordnet, wobei jeweils unterschiedliche Bereiche in den Schutzhüllen ausgenommen sind. Auf diese Weise können in verschiedenen Bereichen unterschiedliche Strukturierungen erzielt werden, z.B. bei Verwendung von Sandkörnern mit abweichender Größe oder Form in einer mehrschrittigen Sandstrahlbehandlung. Unterschiedliche Strukturierungen lassen sich mittels einer Sandstrahlbehandlung weiterhin durch Variation des Drucks und/oder des Winkels erzielen, mit dem der Sandstrahl auf den Roh-Formkörper auftreffen gelassen wird. Auch über eine Variation der Dauer der Sandstrahlbehandlung lassen sich unterschiedliche Strukturierungen erzielen.

Optional können die Strukturierungen auf dem Formkörper in dem Bereich am Ansatz der Greifhilfe, im Bereich der Spitze der Greifhilfe und an den Fingerspitzen sowie optionale weitere Strukturierungen auch durch unterschiedliche Verfahren auf den Formkörper aufgebracht werden. Beispielsweise können die Strukturierungen an den Fingerspitzen und die Strukturierungen im Bereich der Spitze der Greifhilfe mittels einer Sandstrahlbehandlung des Roh-Formkörpers erzeugt werden, während der strukturierte Bereich am Ansatz der Greifhilfe mittels Aufsprühens von Material auf den Roh-Formkörper erzeugt wird. Auf diese Weise werden Strukturierungen mit unterschiedlicher Orientierung (Mulden in der Oberfläche bzw. Erhebungen auf der Oberfläche) erzeugt, die sich zudem auch in ihrer Rauheit unterscheiden. Denn in dieser Ausführungsform weist der Bereich mit strukturierter Oberfläche am Ansatz der Greifhilfe Erhebungen auf der Oberfläche und einen Mittenrauwert Ra von 6.0 bis 9.0 µm auf, während der Bereich mit strukturierter Oberfläche auf der Greifhilfe (insbesondere auf der Spitze der Greifhilfe) Mulden in der Oberfläche und einen Mittenrauwert Ra von 8.0 bis 10 µm aufweist und die Bereiche mit strukturierter Oberfläche an den Fingerspitzen der Hand ebenfalls Mulden in der Oberfläche und einen Mittenrauwert Ra von 6.0 bis 9.0 µm aufweisen.

Optional wird zusätzlich zu der Sandstrahlbehandlung und/oder der Auftragung von zusätzlichem Material auf den Roh-Formkörper noch an weiteren ausgewählten Stellen oder an der gesamten Oberfläche zusätzliches Material aufgesprüht, um eine leichte stellenweise oder vollständige Texturierung zu erhalten, beispielsweise im Bereich der Handflächen des Formkörpers.

Auch überlappende Strukturierungen auf dem Formkörper sind mittels Sandstrahlbehandlung und/oder mittels Aufbringens von Material problemlos zu erzeugen. Dazu wird der Roh-Formkörper in nacheinander erfolgenden Behandlungsschritten mit Schutzhüllen bedeckt, deren Ausnehmungen teilweise an den gleichen Stellen angeordnet sind, insbesondere in etwa den gleichen Mittelpunkt aufweisen, sich jedoch in ihrer Größe unterscheiden. An Stellen, an denen mehrere Schutzhüllen eine Ausnehmung aufwiesen, weist der Formkörper entsprechend stärkere bzw. mehr Strukturierungen auf als an Stellen, an denen nur eine Schutzhülle eine Ausnehmung aufwies. Insbesondere im Bereich der Greifhilfe weisen erfindungsgemäße Formkörper daher überlappende Strukturierungen auf, die sich z.B. in ihrer Art, Tiefe und Verteilung unterscheiden. Insbesondere kann die Greifhilfe dabei durch eine oder mehrere Sandstrahlbehandlungen erzeugte strukturierte Bereiche sowie auch Strukturierungen aufweisen, die durch Auftragen, insbesondere Aufsprühen, von Material erzeugt wurden. Da die Schutzhüllen bei der Sandstrahlbehandlung und/oder beim Aufbringen von Material nicht zu eng am Formkörper anliegen dürfen, entstehen auf der vergleichsweise kleinen Greifhilfe des Formkörpers dabei Bereiche, in denen drei oder mehr verschiedene Strukturierungen überlappen.

Die Erfindung betrifft weiterhin auch die Verwendung des erfindungsgemäßen Formkörpers zur Herstellung eines Handschuhs.

### Genaue Beschreibung der Erfindung

Die Erfindung wird nun mit Bezug auf die Figuren beschrieben, die schematisch
- in Figur 1 einen erfindungsgemäßen Formkörper 1 und
- in Figur 2 eine weitere Ausführungsform eines erfindungsgemäßen Formkörpers 1 zeigen.

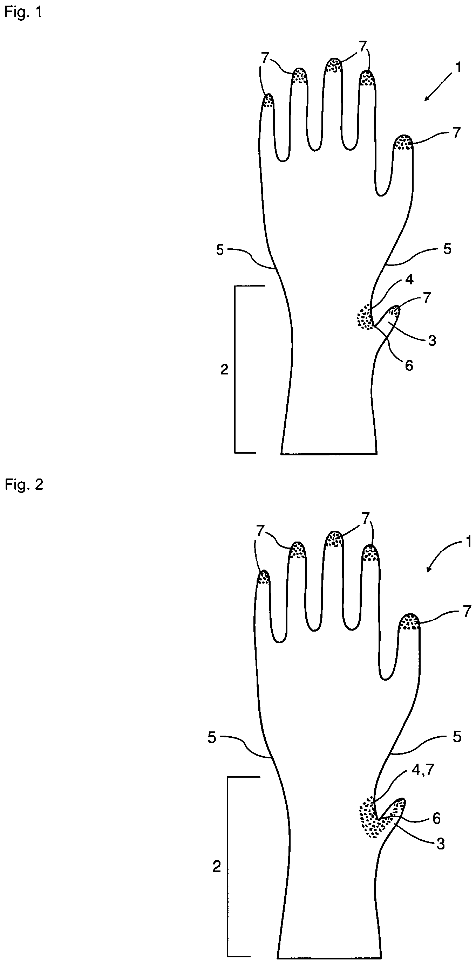

Der in der Figur 1 schematisch dargestellte Formkörper **1** weist die Geometrie einer Hand mit einem daran ansetzenden im Wesentlichen zylindrischen Abschnitt **2** auf. Weiterhin weist der Formkörper **1** eine Greifhilfe **3** auf, die unterhalb des Daumens (d.h. auf dessen von den Fingerspitzen abgewandten Seite) auf der Verlängerung einer der Schmalseiten **5** der Hand in Längsrichtung angeordnet ist.

Am Ansatz **6** der Greifhilfe **3** weist der Formkörper **1** einen Bereich mit strukturierter Oberfläche **4** am auf, der sich in Richtung der Finger der Hand erstreckt und auch als "crotch area" bezeichnet wird. Dieser Bereich mit strukturierter Oberfläche **4** ist stärker strukturiert als andere Bereiche des Formkörpers **1** und insbesondere stärker strukturiert als der unmittelbar an die crotch area angrenzende Bereich, der eine vergleichbare Entfernung zu den Fingerspitzen des Formkörpers **1** oder zu dessen den Fingerspitzen gegenüberliegenden Ende hat.

Für die Zwecke der Erfindung werden die Teile des Formkörpers **1**, die in ihrer Gesamtheit die Geometrie einer Hand aufweisen, entsprechend der vergleichbar geformten Körperteile eines Menschen benannt. Mit "Hand", "Fingern", "Fingerspitzen", "Handfläche", "Handrücken", "Daumen" und "Handgelenk" sind daher keine Körperteile eines Menschen, sondern die jeweiligen vergleichbar geformten Teile des Formkörpers **1** gemeint. Der Formkörper **1** besteht in der gezeigten Ausführungsform aus einem ersten Teil, der die Geometrie einer Hand aufweist, und einem zweiten Teil, der ein an die Hand ansetzender zylindrischer Abschnitt **2** ist und auf dessen Umfang eine Greifhilfe **3** angeordnet ist.

Die Greifhilfe **3** ist ähnlich geformt wie ein Finger des Formkörpers **1**, jedoch kleiner in ihren Ausmaßen und mit einer ausgeprägteren Spitze. Während die Fingerspitzen des Formkörpers 1 deutlich runder geformt sind, weist die Greifhilfe **3** ein spitzeres Ende auf, was den Vorteil hat, dass beim Eintauchen des Formkörpers **1** in das Tauchbad eine kleinere Kontaktfläche besteht, wodurch Luftbläschen besser entweichen können.

Zusätzlich zu dem strukturierten Bereich **4** am Ansatz **6** der Greifhilfe **3** weist der Formkörper **1** auch im Bereich der Spitze der Greifhilfe **3** sowie im Bereich der Fingerspitzen Bereiche mit strukturierter Oberfläche **7** auf. Vorzugsweise sind dabei die Strukturierungen in dem strukturierten Bereich **4** am Ansatz **6** der Greifhilfe **3** Erhebungen, die von der Oberfläche des Formkörpers **1** hervortreten und die durch Auftragen von zusätzlichem Material auf den Formkörper **1** erzeugt wurden, während die Strukturierungen in den strukturierten Bereichen **7** an den Fingerspitzen sowie an der Spitze der Greifhilfe **3** Mulden in der Oberfläche des Formkörpers **1** sind, die durch eine Sandstrahlbehandlung erzeugt wurden.

Besonders bevorzugt weist der Formkörper **1** zusätzlich auf seiner gesamten Oberfläche sog. Mikro-Strukturierungen auf, die durch Auftragen von zusätzlichem Material auf den Formkörper **1** erzeugt wurden und eine geringere Rauheit aufweisen als die Strukturierungen in dem Bereich mit strukturierter Oberfläche **4** am Ansatz **6** der Greifhilfe **3** und die Strukturierungen in den Bereichen mit strukturierter Oberfläche **7** an den Fingerspitzen sowie an der Spitze der Greifhilfe **3**.

Dadurch, dass der Bereich mit strukturierter Oberfläche **4** am Ansatz **6** der Greifhilfe **3** erfindungsgemäß stärkere Strukturierungen aufweist als die daran angrenzenden Bereiche, insbesondere der daran angrenzende Bereich mit gleicher Entfernung zu dem Ende des Formkörpers **1**, das den Fingerspitzen gegenüberliegt, lagert sich beim Eintauchen des Formkörpers **1** in ein Tauchbad mehr Material in dem Bereich mit strukturierter Oberfläche **4** an und durch die erhöhte Rauheit in diesem Bereich wird zudem das Risiko einer Bläschenbildung im Herstellungsprozess der Handschuhe signifikant reduziert.

Die in Figur 2 gezeigte Ausführungsform des erfindungsgemäßen Formkörpers **1** unterscheidet sich dadurch von der in Figur 1 gezeigten Ausführungsform, dass der Formkörper **1** einen Bereich mit strukturierter Oberfläche **4** am Ansatz **6** der Greifhilfe **3** aufweist, der sich einerseits in Richtung der Fingerspitzen der Hand und andererseits in Richtung der Spitze der Greifhilfe **3** erstreckt, wobei der Bereich mit strukturierter Oberfläche **4** am Ansatz **6** der Greifhilfe **3** in den Bereich mit strukturierter Oberfläche **7** an der Greifhilfe **3** übergeht. Gemäß dieser Ausführungsform handelt es sich also bei dem Bereich **4** mit strukturierter Oberfläche am Ansatz **6** der Greifhilfe **3** und dem Bereich **7** mit strukturierter Oberfläche an der Greifhilfe **3** nicht um verschiedene Bereiche, sondern um einen einzigen Bereich, der optional unterschiedliche Strukturierungsarten und/oder -tiefen aufweist.

Wie auch in der in Fig. 1 gezeigten Ausführungsform bildet die Rückseite der Greifhilfe **3** einen parabelförmigen Übergang zu dem zylindrischen Abschnitt **2**, der zur Formgebung der Stulpe dient.

Die in der vorstehenden Beschreibung, in den Ansprüchen und in den Figuren offenbarten Merkmale der Erfindung können sowohl einzeln als auch in beliebigen Kombinationen für die Verwirklichung der Erfindung in ihren verschiedenen Ausführungsformen wesentlich sein.

### Bezugszeichenliste:

- 1: Formkörper
- 2: zylindrischer Abschnitt
- 3: Greifhilfe
- 4: Bereich mit strukturierter Oberfläche am Ansatz der Greifhilfe
- 5: Schmalseiten der Hand (Teil des Formkörpers)
- 6: Ansatz der Greifhilfe
- 7: Bereich mit strukturierter Oberfläche an der Greifhilfe und an den Fingerspitzen des Formkörpers

## Patentansprüche

1. Formkörper (1) zur Herstellung eines Handschuhs, wobei der Formkörper (1) die Geometrie einer Hand mit einem daran ansetzenden im Wesentlichen zylindrischen Abschnitt (2) und mit einer Greifhilfe (3) aufweist, die unterhalb des kleinen Fingers oder unterhalb des Daumens an einer der beiden die Handfläche und den Handrücken verbindenden Schmalseiten (5) der Hand oder alternativ auf der Verlängerung einer dieser Schmalseiten (5) der Hand in Längsrichtung des mit der Hand verbundenen zylinderförmigen Abschnitts (2) angeordnet ist, **dadurch gekennzeichnet, dass** der Formkörper (1) einen Bereich mit strukturierter Oberfläche (4) am Ansatz (6) der Greifhilfe (3) aufweist, der stärker strukturiert ist als andere Bereiche des Formkörpers (1).

2. Formkörper nach Anspruch **1**, **dadurch gekennzeichnet, dass** der Formkörper (1) auch auf der Greifhilfe (3) sowie an zumindest zwei Fingerspitzen der Hand jeweils zumindest einen Bereich mit strukturierter Oberfläche (7) aufweist.

3. Formkörper (1) nach Anspruch 1 oder 2, **dadurch gekennzeichnet, dass** der am Ansatz (6) der Greifhilfe (3) angeordnete Bereich mit strukturierter Oberfläche (4) Strukturierungen aufweist, die sich in ihrer Rauheit von den Strukturierungen auf der Greifhilfe (3) und/oder von den Strukturierungen an den Fingerspitzen unterscheiden.

4. Formkörper (1) nach einem der voranstehenden Ansprüche, **dadurch gekennzeichnet, dass** sich der Bereich mit strukturierter Oberfläche (4) am Ansatz (6) der Greifhilfe (3) in Richtung der Finger der Hand erstreckt, insbesondere um ca. 0,1 bis 3 cm.

5. Formkörper (1) nach einem der voranstehenden Ansprüche, **dadurch gekennzeichnet, dass** der Bereich mit strukturierter Oberfläche (4) am Ansatz (6) der Greifhilfe (3) eine im Vergleich zu einem angrenzenden Bereich des Formkörpers (1) mit gleichem Abstand zu dessen den Fingerspitzen gegenüberliegendem Ende erhöhte Rauheit aufweist.

6. Formkörper (1) nach einem der voranstehenden Ansprüche, **dadurch gekennzeichnet, dass** der Bereich mit strukturierter Oberfläche (4) am Ansatz (6) der Greifhilfe (3) eine ovale oder parabelförmige Fläche bildet.

7. Formkörper (1) nach einem der voranstehenden Ansprüche, **dadurch gekennzeichnet, dass** der Bereich mit strukturierter Oberfläche (4) am Ansatz (6) der Greifhilfe (3) und/oder einer oder mehrere der Bereiche mit strukturierter Oberfläche (7) auf der Greifhilfe (3) oder an den Fingerspitzen der Hand sich überlappende Teilbereiche mit voneinander abweichenden Strukturierungen aufweist.

8. Formkörper (1) nach einem der voranstehenden Ansprüche, **dadurch gekennzeichnet, dass** die Rauheit der Strukturierungen in den Bereichen mit strukturierter Oberfläche (7) an den Fingerspitzen der Hand größer ist als die Rauheit der Strukturierungen in dem Bereich mit strukturierter Oberfläche (4) am Ansatz (6) der Greifhilfe (3).

9. Formkörper (1) nach einem der voranstehenden Ansprüche, **dadurch gekennzeichnet, dass** der Mittenrauwert Ra des Bereichs mit strukturierter Oberfläche (4) am Ansatz (6) der Greifhilfe (3) 6.0 bis 9.0 µm beträgt, während der Mittenrauwert Ra des Bereichs mit strukturierter Oberfläche (7) auf der Greifhilfe (3) 8.0 bis 10.0 µm beträgt, und der Mittenrauwert Ra der Bereiche mit strukturierter Oberfläche (7) an zumindest zwei Fingerspitzen der Hand 6.0 bis 9.0 µm beträgt.

10. Formkörper (1) nach einem der voranstehenden Ansprüche, **dadurch gekennzeichnet, dass** der Bereich mit strukturierter Oberfläche (4) am Ansatz (6) der Greifhilfe (3) auf der der Greifhilfe (3) gegenüberliegenden Seite an einen Bereich angrenzt, der weniger starke oder gar keine Strukturierungen aufweist und dadurch eine geringere Rauheit aufweist als der Bereich mit strukturierter Oberfläche (4) am Ansatz (6) der Greifhilfe (3).

11. Verfahren zur Herstellung eines Formkörpers (1) nach einem der voranstehenden Ansprüche, **dadurch gekennzeichnet, dass** auf einem Roh-Formkörper mit der Geometrie einer Hand mit einem daran ansetzenden im Wesentlichen zylindrischen Abschnitt (2) und mit einer Greifhilfe (3), die unterhalb des kleinen Fingers oder unterhalb des Daumens an einer der beiden die Handfläche und den Handrücken verbindenden Schmalseiten (5) der Hand oder alternativ auf der Verlängerung einer dieser Schmalseiten (5) der Hand in Längsrichtung des mit der Hand verbundenen zylinderförmigen Abschnitts (2) angeordnet ist, ein Bereich mit strukturierter Oberfläche (4) am Ansatz (6) der Greifhilfe (3) erzeugt wird.

12. Verfahren zur Herstellung eines Formkörpers (1) nach Anspruch 11, **dadurch gekennzeichnet, dass** auf dem Roh-Formkörper Bereiche mit strukturierter Oberfläche (7) auf der Greifhilfe (3) und an zumindest zwei Fingerspitzen der Hand erzeugt werden.

13. Verfahren zur Herstellung eines Formkörpers (1) nach Anspruch 12, **dadurch gekennzeichnet, dass** der Bereich mit strukturierter Oberfläche (4) am Ansatz (6) der Greifhilfe (3) und/oder der Bereich mit strukturierter Oberfläche (7) an der Greifhilfe (3) und/oder die Bereiche mit strukturierter Oberfläche (7) an den Fingerspitzen mittels einer Sandstrahlbehandlung des Roh-Formkörpers oder dadurch erzeugt werden, dass zusätzliches Material auf den Bereich bzw. die Bereiche des Roh-Formkörpers aufgesprüht wird.

14. Verfahren nach einem der Ansprüche 12 oder 13, **dadurch gekennzeichnet, dass** die Strukturierungen im Bereich mit strukturierter Oberfläche (4) am Ansatz (6) der Greifhilfe (3) mittels Aufbringens von Material auf den Roh-Formkörper erzeugt werden, und die Strukturierungen in dem Bereich mit strukturierter Oberfläche (7) an der Greifhilfe (3) und in den Bereichen mit strukturierter Oberfläche (7) an den Fingerspitzen mittels einer Sandstrahlbehandlung des Roh-Formkörpers erzeugt werden.

15. Verwendung eines Formkörpers (1) nach einem der Ansprüche 1 bis 10 zur Herstellung eines Handschuhs.

## Claims

1. Former (1) for the production of a glove, the former (1) having the geometry of a hand having an essentially cylindrical section (2) attached thereto and having a gripping aid (3) which is arranged below the little finger or below the thumb at one of the two narrow sides (5) of the hand connecting the palm and the back of the hand or, alternatively, on the extension of one of these narrow sides (5) of the hand in the longitudinal direction of the cylindrical section (2) connected to the hand, **characterized in that** the former (1) has a region having a structured surface (4) at the base (6) of the gripping aid (3), which region is more structured than other regions of the former (1)

2. Former according to claim 1, **characterized in that** the former (1) also has at least one region having a structured surface (7) each on the gripping aid (3) as well as at at least two fingertips of the hand.

3. Former (1) according to claim 1 or 2, **characterized in that** the region having a structured surface (4) arranged at the base (6) of the gripping aid (3) has structures which differ in their roughness from the structures on the gripping aid (3) and/or differ from the structures at the fingertips.

4. Former (1) according to one of the preceding claims, **characterized in that** the region having a structured surface (4) at the base (6) of the gripping aid (3) extends in the direction of the fingers of the hand, in particular by about 0.1 to 3 cm.

5. Former (1) according to one of the preceding claims, **characterized in that** the region having a structured surface (4) at the base (6) of the gripping aid (3) has an increased roughness when compared to an adjacent region of the former (1) having the same distance to its end opposite the fingertips.

6. Former (1) according to one of the preceding claims, **characterized in that** the region having a structured surface (4) at the base (6) of the gripping aid (3) forms an oval or parabolic surface.

7. Former (1) according to one of the preceding claims, **characterized in that** the region having a structured surface (4) at the base (6) of the gripping aid (3) and/or one or more of the regions having a structured surface (7) on the gripping aid (3) or at the fingertips of the hand has overlapping partial regions with differing structuring.

8. Former (1) according to one of the preceding claims, **characterized in that** the roughness of the structuring in the regions having a structured surface (7) at the fingertips of the hand is greater than the roughness of the structuring in the region having a structured surface (4) at the base (6) of the gripping aid (3).

9. Former (1) according to one of the preceding claims, **characterized in that** the average roughness value Ra of the region having a structured surface (4) at the base (6) of the gripping aid (3) is 6.0 to 9.0 µm, whereas the average roughness value Ra of the region having a structured surface (7) on the gripping aid (3) is 8.0 to 10.0 µm, and the average roughness value Ra of the regions having a structured surface (7) on at least two fingertips of the hand is 6.0 to 9.0 µm.

10. Former (1) according to one of the preceding claims, **characterized in that** on the side opposite the gripping aid (3) the region having a structured surface (4) at the base (6) of the gripping aid (3) adjoins a region having less strong or no structuring at all and therefore has a lower roughness than the region having a structured surface (4) at the base (6) of the gripping aid (3).

11. Method for production of a former (1) according to one of the preceding claims, **characterized in that** on a raw former having the geometry of a hand with an essentially cylindrical section (2) attached thereto and having a gripping aid (3) which is arranged below the little finger or below the thumb on one of the two narrow sides (5) of the hand connecting the palm and the back of the hand or alternatively on the extension of one of these narrow sides (5) of the hand in the longitudinal direction of the cylindrical section (2) connected to the hand, a region having a structured surface (4) is generated at the base (6) of the gripping aid (3).

12. Method for production of a former (1) according to Claim 11, **characterized in that** on the raw former regions having a structured surface (7) are generated on the gripping aid (3) and at at least two fingertips of the hand.

13. Method for production of a former (1) according to claim 12, **characterized in that** the region having a structured surface (4) at the base (6) of the gripping aid (3) and/or the region having a structured surface (7) at the gripping aid (3) and/or the regions having a structured surface (7) at the fingertips are generated by a sandblasting treatment of the raw former or **in that** additional material is sprayed onto the region or regions of the raw former, respectively.

14. Method according to one of the claims 12 or 13, **characterized in that** the structuring in the region having a structured surface (4) at the base (6) of the gripping aid (3) is produced by applying material to the raw former, and the structuring in the regions having a structured surface (7) on the gripping aid (3) and in the regions having a structured surface (7) at the fingertips are produced by means of a sandblasting treatment of the raw former.

15. Use of a former (1) according to one of the claims 1 to 10 for the production of a glove.

## Revendications

1. Corps moulé (1) destiné à la fabrication d'un gant, le corps moulé (1) présentant la géométrie d'une main avec un segment sensiblement cylindrique (2) raccordé à celle-ci et avec une aide à la préhension (3) qui est située au-dessous de l'auriculaire ou au-dessous du pouce sur l'un des deux côtés étroits (5) de la main qui relient la paume et le dos de la main ou en variante sur le prolongement de l'un de ces côtés étroits (5) de la main dans le sens longitudinal du segment cylindrique (2) raccordé à la main, **caractérisé en ce que** le corps moulé (1) comporte à l'emplacement (6) de l'aide à la préhension (3) une zone à surface structurée (4) qui est plus fortement structurée que les autres zones du corps moulé (1).

2. Corps moulé (1) selon la revendication 1, **caractérisé en ce que** le corps moulé (1) comporte également sur l'aide à la préhension (3) ainsi qu'au niveau d'au moins deux bouts de doigts de la main chaque fois au moins une zone à surface structurée (7).

3. Corps moulé (1) selon la revendication 1 ou 2, **caractérisé en ce que** la zone à surface structurée (4) située à l'emplacement (6) de l'aide à la préhension (3) comporte des structurations qui diffèrent dans leur rugosité des structurations sur l'aide à la préhension (3) et/ou des structurations au niveau des bouts de doigts.

4. Corps moulé (1) selon l'une quelconque des revendications précédentes, **caractérisé en ce que** la zone à surface structurée (4) à l'emplacement (6) de l'aide à la préhension (3) s'étend, en particulier d'environ 0,1 à 3 cm, en direction des doigts de la main.

5. Corps moulé (1) selon l'une quelconque des revendications précédentes, **caractérisé en ce que** la zone à surface structurée (4) à l'emplacement (6) de l'aide à la préhension (3) présente une rugosité élevée en comparaison d'une zone adjacente du corps moulé (1) à la même distance de l'extrémité opposée aux bouts des doigts de ce dernier.

6. Corps moulé (1) selon l'une quelconque des revendications précédentes, **caractérisé en ce que** la zone à surface structurée (4) à l'emplacement (6) de l'aide à la préhension (3) forme une surface ovale ou parabolique.

7. Corps moulé (1) selon l'une quelconque des revendications précédentes, **caractérisé en ce que** la zone à surface structurée (4) à l'emplacement (6) de l'aide à la préhension (3) et/ou une ou plusieurs des zones à surface structurée (7) sur l'aide à la préhension (3) ou au niveau des bouts des doigts de la main comporte(nt) des zones partielles se chevauchant, à structurations qui diffèrent les unes des autres.

8. Corps moulé (1) selon l'une quelconque des revendications précédentes, **caractérisé en ce que** la rugosité des structurations dans les zones à surface structurée (7) au niveau des bouts des doigts de la main est supérieure à la rugosité des structurations dans la zone à surface structurée (4) à l'emplacement (6) de l'aide à la préhension (3).

9. Corps moulé (1) selon l'une quelconque des revendications précédentes, **caractérisé en ce que** la rugosité moyenne Ra de la zone à surface structurée (4) à l'emplacement (6) de l'aide à la préhension (3) vaut de 6,0 à 9,0 µm, tandis que la rugosité moyenne Ra de la zone à surface structurée (7) sur l'aide à la préhension (3) vaut de 8,0 à 10,0 µm, et la rugosité moyenne Ra des zones à surface structurée (7) au niveau d'au moins deux bouts de doigts de la main vaut de 6,0 à 9,0 µm.

10. Corps moulé (1) selon l'une quelconque des revendications précédentes, **caractérisé en ce que** la zone à surface structurée (4) à l'emplacement (6) de l'aide à la préhension (3) est adjacente, du côté opposé à l'aide à la préhension (3), à une zone qui comporte des structurations moins marquées ou même absentes et de ce fait présente une plus faible rugosité que la zone à surface structurée (4) à l'emplacement (6) de l'aide à la préhension (3).

11. Procédé pour la production d'un corps moulé (1) selon l'une quelconque des revendications précédentes, **caractérisé en ce que** sur un corps moulé brut présentant la géométrie d'une main avec un segment sensiblement cylindrique (2) raccordé à celle-ci et avec une aide à la préhension (3) qui est située au-dessous de l'auriculaire ou au-dessous du pouce sur l'un des deux côtés étroits (5) de la main qui relient la paume et le dos de la main ou en variante sur le prolongement de l'un de ces côtés étroits (5) de la main dans le sens longitudinal du segment partie cylindrique (2) raccordé à la main, est réalisée une zone à surface structurée (4) à l'emplacement (6) de l'aide à la préhension (3).

12. Procédé pour la production d'un corps moulé (1) selon la revendication 11, **caractérisé en ce que** sur le corps moulé brut des zones à surface structurée (7) sont réalisées sur l'aide à la préhension (3) et au niveau d'au moins deux bouts de doigts de la main.

13. Procédé pour la production d'un corps moulé (1) selon la revendication 12, **caractérisé en ce que** la zone à surface structurée (4) à l'emplacement (6) de l'aide à la préhension (3) et/ou la zone à surface structurée (7) au niveau de l'aide à la préhension (3) et/ou les zones à surface structurée (7) au niveau des bouts de doigts sont réalisées au moyen d'un traitement par sablage du corps moulé brut ou par le fait que du matériau supplémentaire est pulvérisé sur la zone ou les zones du corps moulé brut.

14. Procédé selon l'une quelconque des revendications 12 et 13, **caractérisé en ce que** les structurations dans la zone à surface structurée (4) à l'emplacement (6) de l'aide à la préhension (3) sont réalisées par application de matériau sur le corps moulé brut, et les structurations dans la zone à surface structurée (7) au niveau de l'aide à la préhension (3) et dans les zones à surface structurée (7) au niveau des bouts des doigts sont réalisées au moyen d'un traitement par sablage du corps moulé brut.

15. Utilisation d'un corps moulé (1) selon l'une quelconque des revendications 1 à 10 pour la fabrication d'un gant.
